# EUROPEAN PATENT APPLICATION

(11) **EP 1 464 962 A2**
(43) Date of publication of application: **06.10.2004**
(21) Application number: 04251814.2
(22) Date of filing: 26.03.2004
(51) Int. Cl.: G01N 33/84, G01N 33/538

(54) **Colloidal silver-biomolecule complexes**

(30) Priority: 28.03.2003 US 401419
(71) Applicant: Agilent Technologies, Inc., Palo Alto, CA 94306 (US)
(72) Inventor: Jhaoka, Seiji, Campbell, CA 95008 (US)
(74) Representative: Tollett, Ian

(57) **Abstract**

The present invention provides a simple one pot method for preparing colloidal silver-biomolecule complexes. The complexes are formed in solution by mixing a biomolecule with appropriate amounts of a silver salt and a source of halide ions. The mixture is subsequently irradiated with light having a wavelength in the visible region. Silver colloid formation and complex formation occur simultaneously and interdependently. The present invention also provides solutions that include the inventive complexes, kits for preparing these, and methods of using the inventive complexes.

## Description

The present invention relates to a method for preparing a colloidal silver-biomolecule complex. It also relates to kits, solutions and methods employing such complexes.

Silver colloids are microscopic silver particles that are suspended in a liquid medium. Typically, the particles range in size between a few nanometers and several microns. The lower bound is the size at which the particles approach atomic dimensions; beyond the upper bound external forces such as gravity become more important than Brownian motion thereby disrupting the suspension.

Typically, colloidal silver is prepared by chemically reducing a silver salt in aqueous solution. Sodium borohydride (Creighton et al., *J. Chem. Soc. Faraday Trans. II* 75:790, 1975) and sodium citrate (Lee and Meisel, *J. Phys. Chem.* 86:3391, 1982) are the most commonly used reducing agents. Colloidal silver has also been prepared using other methods, e.g., using gamma radiation (Henglein, *J. Phys. Chem.* 84:2461, 1980), by photochemical reduction (Mulvaney et al., *J. Phys. Chem.* 95:7843, 1991), by laser ablation of bulk silver surfaces (Nedderson et al., *Appl. Spectrosc.* 47:1959, 1993), and using UV radiation (Kapoor, *Langmuir* 14:1021, 1998).

As a consequence of their particulate nature and small size, silver colloids exhibit unusual physical and chemical properties. For example, colloidal silver exhibits plasmon resonances that are dependent on the environment, size, shape, and level of aggregation of the particles (Bell and Myrick, *J. Colloid Interface Sci.* 242:300, 2001 and Jin et al., *Science* 294:1901, 2001). In addition molecules that are associated with silver colloids generate enhanced Raman signals - a phenomenon known as surface-enhanced Raman scattering (SERS) *("Surface Enhanced Raman Scattering"* Ed. by Chang and Furtak, Plenum, New York, 1981).

Recently, there has been significant interest in using the unusual properties of silver colloids to detect and/or study molecules (e.g., see Kneipp et al., *J. Molecular Structure* 244:183, 1991; Broderick et al., *Biochemistry* 32:13771, 1993; and Nie and Emory, *Science* 275:1102, 1997). In each of these cases, the silver colloid was prepared in the absence of the molecule of interest, e.g., by conventional chemical reduction of a silver salt. Colloidal silver-molecule complexes were then prepared by contacting the molecule of interest with the pre-formed particles - silver colloid formation and complex formation occurred separately and independently.

The present invention provides a simple one pot method for preparing colloidal silver-biomolecule complexes. The complexes are formed in solution by mixing a biomolecule with appropriate amounts of a silver salt and a source of halide ions. Conventional reducing agents such as sodium borohydride or sodium citrate are not present in the mixture. The mixture is subsequently irradiated with light having one or more wavelengths in the visible region. Surprisingly, silver colloids do not form if the biomolecule is removed from the mixture. In addition, the source of halide ions and visible light irradiation are required. The present invention also provides solutions that include the inventive complexes, kits for preparing these, and methods of using the inventive complexes.

A number of preferred embodiments of the present invention will now be described in conjunction with the accompanying drawings in which:
Figures 1A and 1B illustrate the use of inventive complexes in an assay for detecting and optionally measuring the concentration of biomolecules in a test sample.
Figure 2 illustrates the use of inventive complexes in an assay that uses an array.
Figure 3 illustrates the use of inventive complexes in a competitive binding assay.
Figure 4 il ustrates the use of inventive complexes in a sandwich binding assay.
Figure 5 is a UV-visible absorption spectrum of an inventive colloidal silver-biomolecule complex. The biomolecule used was bovine serum albumin (BSA).
Figure 6 compares UV-visible absorption spectra of inventive colloidal silver-biomolecule complexes. The biomolecules used were poly(guanosine), a synthetic 25-mer of DNA (oligoDNA), and double stranded herring sperm DNA (dsDNA).
Figures 7A, 7B, and 7C compare photographs of solutions that include inventive colloidal silver-biomolecule complexes prepared with different amounts of silver salt.
Figures 8A, 8B, and 8C compare photographs of solutions that include inventive colloidal silver-biomolecule complexes prepared with different amounts of halide ion.
Figures 9A, 9B, and 9C compare photographs of solutions that include inventive colloidal silver-biomolecule complexes prepared with different amounts of biomolecule.
Figure 10 compares UV-visible absorption spectra of inventive colloidal silver-biomolecule complexes. The complexes were prepared with different sources of halide ion, namely zinc chloride, sodium chloride, potassium chloride, and hydrogen chloride.
Figure 11 compares UV-visible absorption spectra of the inventive colloidal silver-biomolecule complexes of Figure 10 after these have been centrifuged and subsequently re-suspended in deionized water.

The present application mentions various patents, scientific articles, and other publications. The contents of each such item are hereby incorporated by reference.

### A. Methods for preparing colloidal silver-biomolecule complexes

The present invention provides a simple one pot method for preparing colloidal silver-biomolecule complexes. The complexes are formed in solution by mixing a biomolecule with appropriate amounts of a silver salt and a source of halide ions. The mixture is subsequently irradiated with light having one or more wavelengths in the visible region. "Appropriate amounts" of a silver salt and a source of halide ions are defined herein as amounts that cause colloidal silver-biomolecule complexes to form in the solution after irradiation.

In general, it is to be understood that the mixing process may be performed using biomolecule, silver salt, and halide ion components that are in solution or in solid form.

In certain embodiments, the inventive method uses three solutions, namely a first solution that includes a biomolecule, a second solution that includes a silver salt, and a third solution that includes halide ions. Preferably the solutions are aqueous. In one embodiment, the mixing process involves simultaneously mixing volumes of the three solutions. In other embodiments, the mixing process involves sequentially mixing volumes of the three solutions. In particular, one may mix a volume of the first solution with a volume of the second solution and then mix the resultant with a volume of the third solution. In each case, the volumes may be mixed by drop-wise addition or by bulk mixing, e.g., in the example above, the second solution may be added drop-wise into the first solution or vice-versa.

The inventive method is not limited to using three solutions, one or two of the components may be in solid form. For example, in one embodiment the mixing process may be performed by simultaneously or sequentially adding a silver salt and a source of halide ions in solid form to a solution that includes a biomolecule.

It is to be understood that the solution may be mixed during, in between, and after each addition (e.g., by stirring, rocking, shaking, etc.). Once the three components have been mixed (or during mixing), the mixture is irradiated with light having one or more wavelengths in the visible region, preferably between 300 - 700 nm. In certain embodiments the one or more wavelengths may fall within a narrower sub-range, e.g., 300 - 500 nm, 500 - 700 nm, 300 - 400 nm, 400 - 500 nm, 500 - 600 nm, or 600 - 700 nm. Any source of irradiation may be used as long as it produces light with one or more wavelengths in the visible range, e.g., without limitation, natural sunlight, incandescent light sources, fluorescent light sources, lasers, etc. The mixture is irradiated for a period of time that is sufficient for colloidal silver-biomolecule complexes to form. In general, this period may range from minutes, to hours, or even days depending in part on the amount of each component within the solution and the nature of the irradiation (e.g., wavelength, intensity, source, etc.). In certain embodiments, the solution is left undisturbed during this period. In certain embodiments, the process may be performed at room temperature. In other embodiments, the mixture may be heated, preferably to a temperature that is lower than the denaturation transition temperature of the biomolecule.

In the examples, the importance of several reaction conditions are analyzed, including the silver salt concentration, the biomolecule concentration, the nature of the biomolecule, the halide ion concentration, the source of halide ion, and the presence of irradiation. The formation of complexes was reduced when irradiation was removed and when the halide ion concentration was decreased. The nature of the halide ion source had no significant effect. Complexes were shown to form in the presence of a variety of biomolecules including polypeptides and both single stranded and double stranded polynucleotides. Surprisingly, the formation of silver colloids was reduced when the biomolecule concentration was decreased.

In general, the inventive complexes may be used directly after preparation and while in an aqueous suspension. Alternatively, the complexes may be stored in the form of an aqueous solution for varying periods of time before being used. In another embodiment, the complex is stored as a dry powder, which may be obtained, e.g., by lyophilization or centrifugation. An aqueous suspension of the complex can then be re-obtained by re-suspending the dry powder in water, preferably deionized water.

### 1. Biomolecules

The inventive method of the present invention may be applied to any biomolecule. As defined herein, the term "biomolecule" encompasses any molecule that comprises a polypeptide, a polynucleotide, or a combination thereof.

As defined herein, a "polypeptide" is a polymer of amino acids. The terms "polypeptide", "protein", and "peptide", may be used interchangeably. Typically a polypeptide comprises a string of at least two amino acids linked together by peptide bonds. Polypeptides of the present invention may contain naturally occurring amino acids and/or non-naturally occurring amino acids (i.e., amino acids that do not occur in nature but that can be incorporated into a polypeptide chain). Also, one or more of the amino acids in an inventive polypeptide may be modified, for example, by the addition of a chemical entity such as a carbohydrate group, a phosphate group, a farnesyl group, an isofarnesyl group, a fatty acid group, a linker for conjugation, functionalization, or other modification, etc.

In certain embodiments one or more of the amino acids may be covalently linked with a fluorescent label. Fluorescent labels of interest include phycoerythin, coumarin and its derivatives, e.g., 7-amino-4-methylcoumarin, aminocoumarin, bodipy dyes, such as BODIPY™ FL, cascade blue, fluorescein and its derivatives, e.g., fluorescein isothiocyanate, Oregon green, rhodamine dyes, e.g., Texas red, tetramethylrhodamine, eosins and erythrosins, cyanine dyes, e.g., Cy3 and Cy5, macrocyclic chelates of lanthanide ions, e.g., QUANTUM DYE™, fluorescent energy transfer dyes, such as thiazole orange-ethidium heterodimer, TOTAB, etc.

Without limitation, exemplary polypeptides that may be included in a biomolecule of the present invention include antibodies and their antigens. As defined herein, a polypeptide "antigen" encompasses any polypeptide that is recognized by an antibody including haptens of an antigen that include a single isolated epitope and fragments of an antigen that include one or more epitopes. Other exemplary polypeptides that may be used are polypeptide ligands or receptors from an affinity pair. Exemplary affinity pairs include biotin/streptavidin, biotin/avidin, digoxigenin/anti-digoxigenin, FK506/FK506-binding protein (FKBP), rapamycin/FKBP, cyclophilin/cyclosporin, and glutathione/glutathione transferase pairs. A variety of other suitable antibody/antigen and ligand/receptor pairs are described by Kessler pp. 105-152 of *"Advances in Mutagenesis"* Ed. by Kessler, Springer-Verlag, 1990 and further in *"Affinity Chromatography: Methods and Protocols (Methods in Molecular Biology)"* Ed. by Pascal Baillon, Humana Press, 2000 and *"Immobilized Affinity Ligand Techniques"* by Hermanson et al, Academic Press, 1992. Yet other exemplary polypeptides may comprise a fluorescent protein, e.g., a member of the family of fluorescent proteins such as green fluorescent protein, etc. as described in Matz et al., *Bioessays* 24:953, 2002. The use of polypeptides that include a chemiluminescent protein (e.g., alkaline phosphatase, horseradish peroxidase, and the like) is also encompassed.

As defined herein, a "polynucleotide" is a polymer of nucleotides. The terms "polynucleotide", "nucleic acid", and "oligonucleotide", may be used interchangeably. Typically, a polynucleotide comprises at least two nucleotides linked together by phosphodiester bonds. DNA and RNA are exemplary oligonucleotides of the present invention. In general, the polynucleotides may be single stranded or double stranded. In certain embodiments, the polynucleotides may contain naturally occurring nucleotides (i.e., nucleotides that include the bases adenine, thymine, cytosine, guanine, or uracil). In certain embodiments, the polynucleotides may include modified nucleotides (e.g., without limitation, nucleotides that include the bases 2-aminoadenine, 2-thiothymine, 3-methyladenine, 5-propynylcytosine, 5-propynyluracil, 5-bromouracil, 5-fluorouracil, 5-iodouracil, 5-methylcytosine, 7-deazaadenine, 7-deazaguanine, 8-oxoadenine, 8-oxoguanine, O(6)-methylguanine, or 2-thiocytosine). Alternatively or additionally, the oligonucleotides may include modified sugars (e.g., 2'-fluororibose, arabinose, hexose, and riboses with a 2'-O, 4'-C-methylene bridge) and/or modified phosphate groups (e.g., phosphorothioates and 5'-N-phosphoramidite linkages). Without limitation, the present invention encompasses the use of biomolecules that include peptide nucleic acids (PNAs), locked nucleic acid (LNAs), and unstructured nucleic acids (UNAs).

Also, one or more of the nucleotides in an inventive polynucleotide may be modified, for example, by the addition of a chemical entity such as a linker for conjugation, functionalization, or other modification, etc. In certain embodiments one or more of the nucleotides may be covalently linked with a fluorescent label.

### 2. Silver salts

The inventive method may be used with any water soluble silver salt. As defined herein, a "water soluble silver salt" is a silver salt with a water solubility of at least 0.1 g/l at room temperature. Without limitation, the silver salt may be silver acetate, silver benzoate, silver bromate, silver chlorate, silver perchlorate, silver chlorite, silver fluoride, silver lactate, silver levunilate, silver permanganate, silver nitrate, silver nitrite, silver propionate, silver sulfate, or silver tartrate. The specific use of silver acetate is described in the examples. In certain embodiments, a mixture of two or more silver salts may be used in preparing an inventive complex. It will be appreciated and it is to be understood that the term "silver salt" as used herein encompasses hydrates of silver salts in addition to dehydrated silver salts.

In certain embodiments, the amount of silver salt that is added to the mixture is determined from the amount of biomolecule that is being added to the mixture. In preferred embodiments, the weight ratio of silver salt to biomolecule added to the mixture is between about 0.1 and 100. In other embodiments the ratio is between about 0.1 and 50, preferably between about 1 and 25. The weight ratio is determined by calculating the amount (in grams) of silver salt that has been added to the mixture and dividing it by the amount (in grams) of biomolecule that has been added to the mixture.

### 3. Source of halide ions

The inventive method of the present invention may be used with any water soluble source of halide ions. As defined herein, a "water soluble source of halide ions" is a source of halide ions with a water solubility of at least 0.1 g/l at room temperature. Without limitation, preferred sources of halide ions are metal halides. It is to be understood and will be appreciated by one of ordinary skill in the art that all metal chlorides, metal bromides, and metal iodides except for those of silver, lead (II), and mercury (I) are considered water-soluble sources of halide ions for the purposes of the present invention. In certain preferred embodiments, the metal halide may be a Group IA, Group IIA, or transition metal chloride, bromide, or iodide. It will be appreciated that the term "metal halide" as used herein encompasses hydrates of metal halides in addition to dehydrated metal halides. The specific use of zinc chloride, sodium chloride, and potassium chloride is described in the examples.

In addition, it is to be understood that the present invention is not limited to using metal halides and that any source of halide ion may be used as long as it is water soluble, e.g., an aqueous solution of an acid halide such as hydrochloric acid could equally be used. In certain embodiments, a mixture of two or more sources of halide ions may be used in preparing an inventive complex.

In certain embodiments, the amount of the source of halide ion that is added to the mixture is determined from the amount of silver ion that is being added to the mixture. In certain embodiments, the molar ratio of halide ion to silver ion added to the mixture is between about 0.0001 and 1. In other embodiments the ratio is between about 0.001 and 1, preferably between 0.01 and 1, more preferably between about 0.01 and 0.1. The molar ratio is determined by calculating the amount (in moles) of halide ion that has been added to the mixture and dividing it by the amount (in moles) of silver ion that has been added to the mixture.

### B. Methods for defecting colloidal silver-biomolecule complexes

Colloidal silver-biomolecule complexes may be detected using any one of a variety of known techniques. In certain embodiments the complexes are detected using microscopy, e.g., without limitation, light microscopy, atomic force microscopy, or electron microscopy. In general, any form of microscopy that can visualize the complexes may be used.

Alternatively or additionally, the complexes may be detected by relying on the strong optical absorption that is exhibited by silver colloids in the visible portion of the spectrum. The strong absorption results from excitation of characteristic surface plasmon resonance or interband transitions. The Mie theory of absorption, which explains much of the fundamental characteristics of colloidal particles, including silver colloids, was published in 1908 (Mie, *Ann. Phys.* 25:377, 1908). A review of the optical properties of colloids can be found in Bohran and Huffman, *"Absorption and Scattering of Light by Small Particles",* Wiley and Sons, New York, 1983. In general, the optical resonances may be detected using a spectrophotometer or simply using the naked eye (e.g., by monitoring a change in color). As described in greater detail in the examples, the complexes that are prepared according to the inventive methods absorb light in the visible region with a maximum (λₘₐₓ) in the range of about 420 nm to about 500 nm. In certain embodiments λₘₐₓ has been found to occur in sub-ranges, e.g., between about 430 - 450 nm, 440 - 460 nm, 450 - 470 nm, 460 - 480 nm, or 470 - 490 nm.

In certain embodiments, the formation of colloidal silver-biomolecule complexes may be further confirmed by centrifuging the solution, discarding the supernatant, and then re-suspending the resulting colloidal pellet. Any biomolecule that is not associated with the colloidal silver will be discarded in the supernatant. The re-suspended solution is then re-investigated, e.g., by microscopy or optical spectroscopy in order to determine whether the biomolecule is still present. In certain embodiments one might also analyze the composition of the supernatant. Without limitation, the presence of polynucleotides in either the supernatant or the re-suspended solution can be determined by monitoring for a characteristic UV absorption (λₘₐₓ of about 260 nm). Similarly, the presence of polypeptides can be assessed by monitoring for a characteristic UV absorption (λₘₐₓ of about 280 nm).

As mentioned in the background, molecules that are in close proximity to silver colloids (e.g., biomolecules that are adsorbed on the surface of a silver colloid as in the present inventive complexes) exhibit Raman signals that are up to six orders of magnitude greater than in the absence of the colloid. This phenomenon commonly called surface-enhanced Raman scattering or SERS has been reviewed in detail (see Schatz, *Acc. Chem. Res.* 17:370, 1984; Moskovits, *Rev. Mod. Phys.* 57:783, 1985; and Otto et al., *J. Phys. Condens. Matter* 4:1143, 1992). In certain embodiments, the formation of complexes may be detected by measuring the enhancement in Raman scattering from the biomolecules in the complexes. It will be appreciated that SERS signals may be detected from any component of the biomolecule including a polynucleotide component, a polypeptide component, a fluorescent label, etc.

### C. Kits

The present invention also provides kits for preparing solutions of inventive complexes. In general, the kit may include a first container means containing an amount of a silver salt, and a second container means containing an amount of a source of halide ions, wherein the silver salt and halide ions form colloidal silver-biomolecule complexes in solution when mixed in appropriate amounts with a biomolecule.

The silver salt may be any water soluble silver salt including silver acetate, silver benzoate, silver bromate, silver chlorate, silver perchlorate, silver chlorite, silver fluoride, silver lactate, silver levunilate, silver permanganate, silver nitrate, silver nitrite, silver propionate, silver sulfate, silver tartrate, or a hydrate thereof. The specific use of silver acetate is described in the examples.

The source of halide ions may be any water soluble source of halide ions. Without limitation, preferred sources of halide ions are metal halides. It is to be understood and will be appreciated by one of ordinary skill in the art that all metal chlorides, metal bromides, and metal iodides except for those of silver, lead (II), and mercury (I) are considered water-soluble sources of halide ions for the purposes of the present invention. In certain preferred embodiments, the metal halide may be a Group IA, Group IIA, or transition metal chloride, bromide, or iodide. It will be appreciated that the term "metal halide" as used herein encompasses hydrates of metal halides in addition to dehydrated metal halides. The specific use of zinc chloride, sodium chloride, and potassium chloride is described in the examples.

In addition, it is to be understood that the present invention is not limited to using metal halides and that any source of halide ion may be used as long as it is water soluble, e.g., an aqueous solution of an acid halide such as hydrochloric acid could equally be used. Further, the first and second container means may include the silver salt and source of halide ions in solution or in solid form.

### D. Methods for using colloidal silver-biomolecule complexes

The present invention further provides a variety of uses for the complexes of the present invention. The following describes non-limiting examples of these uses. It is to be understood that the invention is in no way limited to these uses and encompasses all uses of the inventive complexes.

### 1. Detecting and measuring the concentration of biomolecules

As described in the examples, the formation of silver colloids is reduced when the silver salt and source of halide ions are mixed in the presence of a reduced amount of biomolecule. In certain embodiments, this aspect of the invention may be used to detect and measure the concentrations of biomolecules.

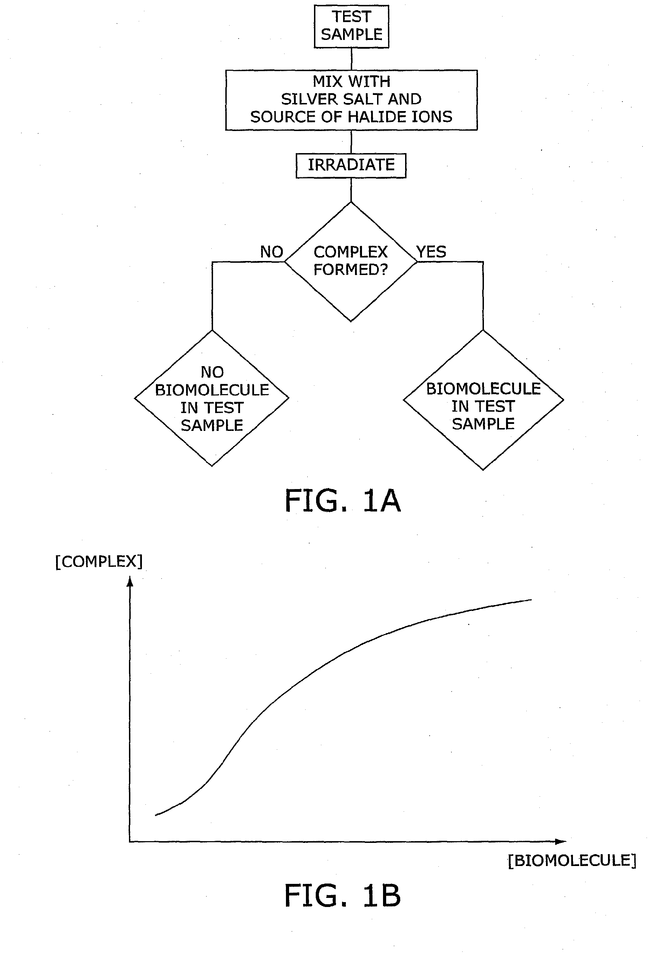

Such a method is illustrated schematically in the flow chart of Figure 1A. As shown, a test sample is provided that may or may not include a biomolecule. The test sample is mixed with a silver salt and a source of halide ions as described previously. The mixture is then irradiated. If a biomolecule is present in the test sample then colloidal silver and complexes should form. The colloidal silver and complexes formed can be detected using any one of the methods described herein, e.g., using the naked eye, microscopy, optical spectroscopy, SERS, etc. If no biomolecule was present in the test sample then no silver colloids should form. This simple one pot test provides a quick and easy way of detecting the presence of biomolecules.

In addition, the level of complex formation will be related to the concentration of biomolecule in the original sample. This is illustrated in greater detail in the examples. In order to determine the concentration of a biomolecule in a sample one might therefore compare the level detected in the test sample with the level that is detected when a known calibration amount of the same biomolecule is used in a separate preparation. In certain embodiments, the level of complex formation may be compared with the levels that are detected with a plurality of different calibration amounts. This is illustrated in Figure 1B.

### 2. Assays itsinz arrays

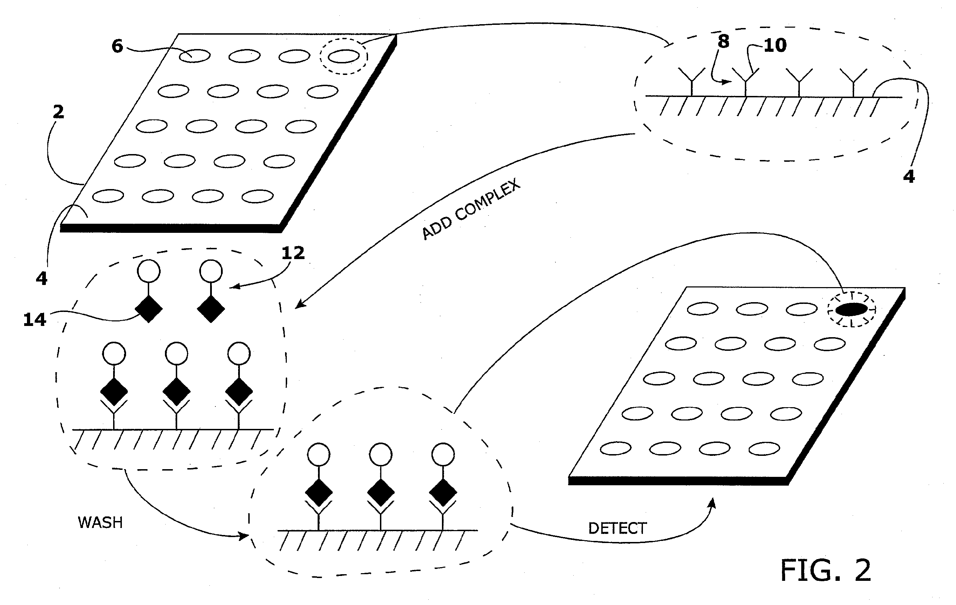

In certain embodiments, the inventive complexes may be used as labeled analytes in an assay that uses an array, e.g., a microarray, a multiwell microtiter plate, etc. As illustrated in Figure 2, according to such embodiments, one or more inventive colloidal silver-target biomolecule complexes are first provided. In preferred embodiments, these are prepared by providing a sample that includes one or more biomolecules and then preparing inventive complexes of these according to the methods described herein. A substrate (2) that is associated on its surface (4) with an array of separate and different molecular elements (6) is then provided. In general, the molecules (8) within different elements (6) of the array include binding complements (10) for different biomolecules (e.g., different complementary polynucleotides). The substrate (2) is then contacted with the colloidal silver-target biomolecule complexes (12) so that the binding complements (10) in the array can interact and bind with biomolecules (14) within the complexes (12). Complexes (12) that are not associated with the substrate (2) are then removed by washing. The location of substrate-associated complexes (12) is then identified (e.g., using any of the methods described herein). The nature of the one or more biomolecules (14) in the sample is then determined based on the step of identifying and prior knowledge of the microarray composition.

In general, the molecular elements of the array comprise molecules that are preferably stably associated with the surface of the substrate. By stably associated is meant that the molecules maintain their position relative to the substrate under binding and washing conditions. As such, the molecules can be covalently or non-covalently stably associated with the substrate surface. A variety of methods are known in the art for covalently or non-covalently associating polynucleotides, antigens, antibodies, ligands, receptors, small molecules, etc. on a variety of surfaces. For example, polynucleotide arrays are discussed in, e.g., Heller, *Annu. Rev. Biomed. Eng.* 4:129, 2002 and *"Current Protocols in Molecular Biology",* Ed. by Ausubel et al., Wiley, NY, 1993; polypeptide arrays are discussed in, e.g., MacBeath, *Nat. Genet.* 32S:526, 2002; Templin et al., *Trends Biotechnol.* 20:160, 2002; Zhu and Snyder, *Curr. Opin. Chem. Biol.* 5:40,2001; MacBeath and Schreiber, *Science* 289:1760, 2000; and small molecule arrays are discussed in, e.g., Lam and Renil, *Curr. Opin. Chem. Biol.* 6:353, 2002 and Falsey et al., *Bioconjug. Chem.* 12:346, 2001.

The substrates upon which the arrays are preferably presented in the subject assays may take a variety of configurations ranging from simple to complex, depending on the intended use of the array. Thus, the substrate could have an overall slide or plate configuration, such as a rectangular or disc configuration, where an overall rectangular configuration, as found in standard microtiter plates and microscope slides, is preferred. The substrates of the arrays may be fabricated from a variety of materials. The materials from which the substrate is fabricated should ideally exhibit a low level of non-specific binding with inventive complexes. In many situations, it will also be preferable to employ a material that is transparent to visible and/or UV light. Specific materials of interest include: glass; plastics, e.g., polytetrafluoroethylene, polypropylene, polystyrene, polycarbonate, blends thereof, and the like; metals, e.g. gold, platinum, and the like; etc. The surface on which the pattern of molecular elements is presented may be modified with one or more different layers of compounds that serve to modulate the properties of the surface in a desirable manner.

The assays of the subject invention may be performed using well known technologies, e.g., contacting with inventive complexes in a suitable container means, under a coverslip, etc., or may be incorporated into a structure that provides for ease of analysis, high throughput, or other advantages, such as in a biochip format, a multiwell format and the like. For example, the subject arrays could be incorporated into a biochip type device in which one has a substantially rectangular shaped cartridge comprising fluid entry and exit ports and a space bounded on the top and bottom by substantially planar rectangular surfaces, wherein the array is present on one of the top and bottom surfaces.

Alternatively, the subject arrays may be incorporated into a high throughput or multiwell device, wherein each array is bounded by raised walls in a manner sufficient to form a reaction container wherein the array is the bottom surface of the container. Generally such devices comprise a plurality of reaction chambers, each of which contains the array on the bottom surface of the reaction chamber.

### 3. Competitive binding assays for identifying the presence of target analytes

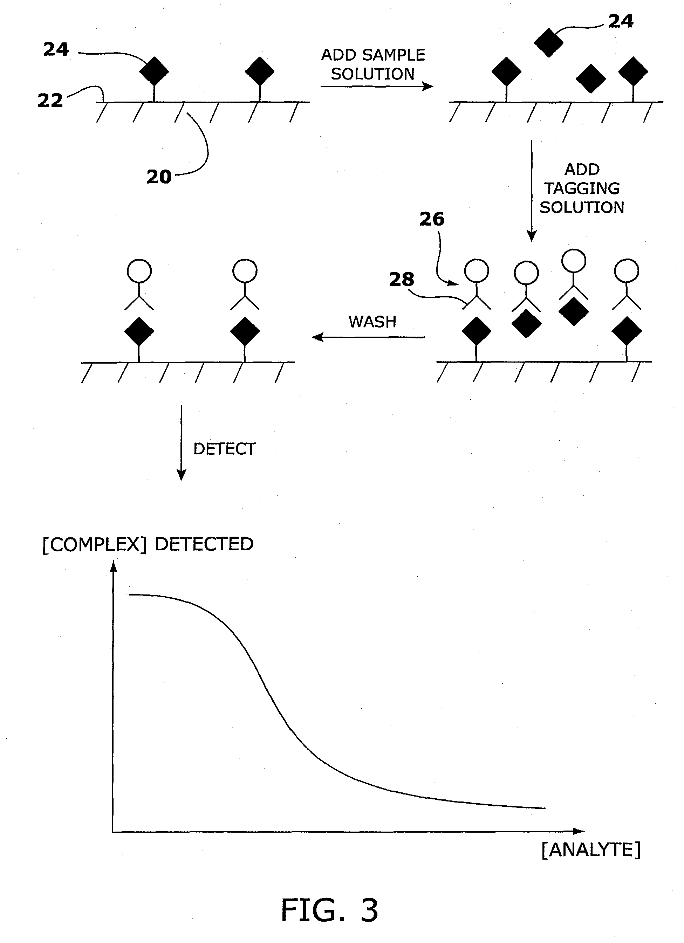

In certain embodiments, the inventive complexes may be used in competitive binding assays for identifying the presence of a target analyte within a sample. Figure 3 schematically illustrates the steps that may be used in a competitive binding assay. According to such an embodiment, a substrate (20) is provided that is associated on its surface (22) with a target analyte (24), e.g., without limitation, an antigen of interest. The substrate (20) is then contacted with a sample solution that may or may not include the target analyte (24). A tagging solution that includes a colloidal silver-biomolecule complex (26) is then added to the sample solution. The biomolecule in the complex (26) is chosen to include a binding complement (28) for the target analyte (24), e.g., without limitation an antibody for a target antigen. Once the tagging solution has been added to the sample solution, the complexes (26) associate with target analytes (24) via the binding complement (28). The target analytes (24) on the surface (22) and the target analytes (24) that were added via the sample solution compete for the complexes (26). Target analytes (24) and complexes (26) that are not associated with the substrate (20) are subsequently removed by washing, leaving the complexes (26) that have become associated with target analytes (24) on the surface (22). The level of complex (26) that remains associated with the substrate (20) is then detected (e.g., using any of the methods described herein) and compared with the level of substrate-associated complex that is detected when the process is repeated without contacting the substrate with the sample solution. Based on this comparison one can readily determine whether the target analyte was present in the sample or not (i.e., if the level of substrate-associated complex decreases when the sample solution is added, then the sample solution includes an amount of the target analyte). In general, the relative amounts of a target analyte that are present in different samples are easily determined by comparing the level of decrease between samples.

In certain embodiments, one may repeat the process with one or more calibration samples that each include a known amount of the target analyte. A standard calibration curve can then be constructed that correlates the concentration of target analyte in the sample with the decrease in the level of substrate-associated complex (Figure 3). Unknown samples can then be tested and accurate concentrations of target analyte determined. It will be readily recognized that inventive competitive binding assays are not limited to antigen analytes and that they can be applied to any form of analyte in combination with a binding complement that can be included within a biomolecule, e.g., without limitation, antibodies, ligands, receptors, complementary polynucleotides, etc. For a general review of competitive binding assays, see, for example, *"Principles of Competitive Protein-Binding Assays",* Ed. by Odell and Franchimont, Wiley, NY, 1982; *"Current Protocols in Immunology",* Ed. by Coligan et al., Wiley, NY, 1994; and *"Current Protocols in Protein Science",* Ed. by Coligan et al., Wiley, NY, 1995.

### 4. Sandwich binding assays for identifying the presence of target analytes

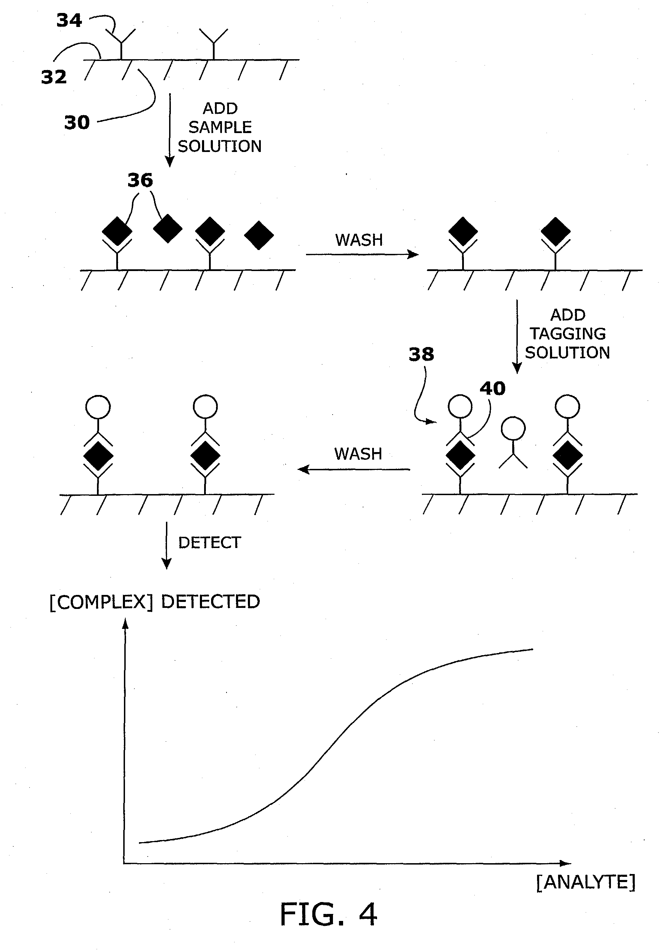

In certain embodiments, the inventive complexes may be used in sandwich binding assays for identifying the presence of target analytes within a sample. Figure 4 schematically illustrates the steps that may be used in a competitive binding assay. According to such an embodiment, a substrate (30) is provided that is associated on its surface (32) with a molecule that includes a first binding complement (34) for a target analyte, e.g., without limitation, a first antibody for a target antigen. The substrate (30) is then contacted with a sample solution that may or may not include the target analyte (36). If target analyte (36) is present in the added sample solution it will interact and bind with the first binding complement (34). Unbound target analytes (36) are then removed by washing. A tagging solution that includes a colloidal silver-biomolecule complex (38) is then added to the sample solution. The biomolecule in the complex (38) is chosen to include a second binding complement (40) for the target analyte (36), e.g., without limitation a second antibody for a target antigen. Once the tagging solution has been added to the sample solution, the complexes (38) associate with target analytes (36) via the second binding complement (40) to form a "sandwich". If no target analytes (36) were present in the sample solution, then the complexes (38) are unable to bind (i.e., no "sandwich" will form). Complexes (38) that are not associated with the substrate (30) are subsequently removed by washing, leaving the complexes (38) that have become associated with target analytes (36) on the surface (32). The level of complex (38) that remains associated with the substrate (30) is then detected (e.g., using any of the methods described herein) and compared with the level of substrate-associated complex that is detected when the process is repeated without contacting the substrate with the sample solution. Based on this comparison one can readily determine whether the target analyte was present in the sample or not (i.e., if the level of substrate-associated complex increases when the sample solution is added, then the sample solution includes an amount of the target analyte). In general, the relative amounts of a target analyte that are present in different samples are easily determined by comparing the level of increase between samples.

In certain embodiments, one may repeat the process with one or more calibration samples that each include a known amount of the target analyte. A standard calibration curve can then be constructed that correlates the concentration of target analyte in the sample with the increase in the level of substrate-associated complex (Figure 4). Unknown samples can then be tested and accurate concentrations of target analyte determined. It will be readily recognized that inventive sandwich binding assays are not limited to antigen analytes and that they can be applied to any form of analyte in combination with a binding complement that can be included within a biomolecule, e.g., without limitation, antibodies, multivalent ligands, receptors, complementary polynucleotides, etc. For a general review of sandwich binding assays, see, for example, *"Current Protocols in Immunology",* Ed. by Coligan et al., Wiley, NY, 1994 and *"Current Protocols in Protein Science",* Ed. by Coligan et al., Wiley, NY, 1995.

### 5. Surface-enhanced Raman scattering (SERS)

In certain embodiments, surface-enhanced Raman scattering (SERS) may be used to study and/or characterize biomolecules that are present within inventive complexes. In other embodiments, SERS may be used to study a molecule that is subsequently associated with an inventive complex, e.g., by binding with the biomolecule component of the complex. Methods and devices for obtaining SERS spectra are well known and have been described in the art (e.g., Schatz, *Acc. Chem. Res.* 17:370, 1984; Moskovits, *Rev. Mod Phys.* 57:783, 1985; and Otto et al., *J. Phys. Condens. Matter* 4:1143, 1992).

As is well known in the art, the SERS phenomenon is manifested as unusually intense Raman signals from molecules that are associated with (or in close proximity to) certain metal surfaces including silver colloid surfaces ("*Surface Enhanced Raman Scattering"* Ed. by Chang and Furtak, Plenum, New York, 1981). In general, the strong enhancement allows (a) the registration of enhanced Raman spectra of molecules in concentration ranges that are close to those necessary for "normal" Raman scattering in solution and/or (b) the registration of normal Raman spectra of molecules in concentration ranges that are three to six fold smaller than necessary for "normal" Raman scattering in solution.

Early investigators measured SERS spectra from a variety of small inorganic and organic molecules that had been adsorbed on silver colloids (e.g., Creighton et al., *J. Chem. Soc. Faraday Trans. II* 75:790, 1979; von Raben et al., *Chem. Phys. Lett.* 79:465, 1981; and Suh et al., *J. Phys. Chem.* 87:1540, 1983). More recently, it has been shown that SERS can be used as a technique for detecting and identifying biomolecules (Cotton, *J. Raman Spect.* 23:729, 1991). In particular, SERS has been used with silver colloids as a method for analyzing polynucleotides (Kneipp et al., *J. Molecular Structure* 244:183, 1991); for analyzing polypeptides (Broderick et al., *Biochemistry* 32:13771, 1993); for single molecule detection (Kneipp et al., *Phys. Rev. Lett.* 78:1667, 1997 and Nie and Emory, *Science* 275:1102, 1997); and for detecting and identifying single base differences in double stranded DNA fragments (Chumanov et al., *Proceedings SPIE,* Vol. 3608, 1999).

### 6. Optical tweezers

In certain embodiments, inventive complexes may be manipulated using optical tweezers, e.g., to form assemblies or arrays of complexes on a surface. Optical tweezers are instruments that can manipulate microscopic objects using the force exerted by light. Objects ranging in size between a few nanometers and several microns have been successfully manipulated with optical tweezers. The objects are attracted to and trapped near the waist of a laser beam that has been focused through a microscope objective.

In general, trapping of metallic objects is more difficult than trapping of dielectric objects such as such as polystyrene or silica beads. Despite this, trapping of 36 nm gold particles has been demonstrated (Svoboda and Block, *Opt. Lett.* 19:930, 1994) and trapping of larger metallic particles has been achieved using a donut shaped TEM^{*}₀₁ Laguerre-Gaussian mode (Sato et al., *Opt. Lett.* 19:1807, 1994; O'Neil and Padgett, *Optics Comm.* 185:139, 2000; and Gu and Morrish, *J. Appl. Physics* 91:1606, 2002) and a single Gaussian beam focused near the bottom of the particle (Furukawa and Yamaguchi, *Opt. Lett.* 23:216, 1998).

In general, optical trapping can be done using lasers over a wide range of wavelengths. Visible lasers, such as the HeNe laser have the advantage that they can be easily seen, and so are relatively easy to align. Infrared lasers such as Nd:YAG or semiconductor lasers with wavelengths around 850 nm may also be used. The construction and operation of optical tweezers are well documented in the literature. For example, Smith et al., *Am. J. Phys.* 67:26, 1999 provides a detailed description of the components, construction and trapping procedure of an optical tweezer device with a single optical trap. In certain embodiments, optical tweezers that include two or more optical traps that allow for the independent manipulation of two or more objects (e.g., two complexes) may be used. As is well known in the art, dual traps can be constructed by combining two beams of the same wavelength and opposite polarization that travel together without interference through the tweezer apparatus to the trapping plane. The beams may, for example, be combined using a polarizing beam-splitting cube (PBS). Adjustable kinematic mirrors corresponding to each beam before they combine can be used to deflect the beams and thereby steer the traps independently. For details on the construction and operation of optical tweezers comprising dual optical traps see, for example, Mammen et al., *Chem Biol.* 3:757, 1996 and Harada et al., *Biophys. J.* 76:709, 1999. For details on the construction and operation of optical tweezers comprising more than two optical traps see, for example, U.S. Patent No. 6,055,106 to Grier; Visscher et al., *Cytometry* 14:105, 1993; Visscher et al., *J. Select. Topics Quantum Electron.* 2:1066, 1996; Dufresne and Grier, *Rev. Sci. Instr.* 69:1974, 1998; and Friedman et al., *Opt. Lett.* 25:1762, 2000. In addition, Arryx Inc. of Chicago sells systems that have been used to generate two- and three-dimensional patterns of traps for both metallic and dielectric objects.

In certain embodiments, single, dual, or multiple optical traps may be used to place one or more inventive complexes at specific locations on a surface, e.g., a glass or metal surface. In certain embodiments, the surface may include a coating that may or may not be patterned. Exemplary coatings include those that associate with silver colloids, e.g., coatings of polylysine, organosilane compounds, or thiol compounds (Freeman et al., *Science* 267:1629, 1995 and Chumanov et al., *J. Phys. Chem.* 99:9466, 1995). Other exemplary coatings may include a binding complement for a biomolecule within an inventive complex (e.g., an antigen for an antibody, a ligand for a receptor, a complementary polynucleotide, etc.).

In one embodiment, the optical trap(s) are used to place inventive complexes that include different biomolecules in different elements of an array. In certain embodiments, these inventive arrays are then used to probe for test samples of interest. For example, in one embodiment, test samples (e.g., a plurality of polynucleotides) are contacted with an array. The locations of binding events within the array can then be determined by detecting surface-enhanced Raman scattering (SERS) from bound test samples. Additionally or alternatively, one may take advantage of changes that occur in the SERS signal of biomolecules within the array when these are bound by a test sample (e.g., as described in U.S. Patent No. 6,376,177 to Poponin).

In other embodiments, the optical trap(s) are used to produce assemblies of inventive complexes. For example, two different inventive complexes that each include one member of a pair of binding complements (e.g., an antigen/antibody pair, a ligand/receptor pair, or two complementary polynucleotides) are trapped separately and then brought into close proximity so that the binding complements can bind. Alternatively, two or more inventive complexes are brought into close proximity and then linked indirectly, e.g., via a freely diffusing multivalent linker that includes two or more binding complements (Storhoff et al., *J. Am. Chem. Soc.* 122:4640, 2000). It will be appreciated that larger and more complex assemblies employing more than two of the same or different inventive complexes may be constructed using optical tweezers.

In certain embodiments, an assembly of inventive complexes may be used as a seed for additional structures. In particular, the process of silver deposition on a polynucleotide strand to form nanowires has been successfully demonstrated (Braun et al., *Nature,* 391:775, 1998). The process involves three main steps. The first step involves exchanging ions in the vicinity of the polynucleotide with silver ions and thereby forming polynucleotide-silver ion complexes. This is followed by the deposition of silver on the polynucleotide strands to form nanometer sized aggregates. The final step involves developing the silver, much as in the standard photographic process using an acidic solution of hydroquinone and silver ions under low light conditions.

### Examples

Features of the present invention will become more apparent from the following description of certain exemplary embodiments thereof.

### Example 1 - Preparing complexes using a polypeptide

The following describes the preparation and characterization of complexes of a polypeptide, namely bovine serum albumin (BSA). The solutions listed in Table 1 were first prepared:

**TABLE 1**

| **Solution** | **Ingredient** | **Concentration (w/v)** |
|---|---|---|
| 1 | silver acetate | 0.2 % |
| 2 | BSA¹ | 1% |
| 3 | zinc chloride | 1 % |

| | | |
|---|---|---|
| ¹Bovine serum albumin from Sigma-Aldrich. | | |

500 µl of solution 1 was added to 1 ml of deionized water in a 1.7 ml Eppendorf tube. 5 µl of solution 2 was then added to the tube and the tube was vortexed to mix the ingredients. Finally, 5 µl of solution 3 was added to the tube, the resulting mixture was vortexed, and then subjected to ambient irradiation of fluorescence light (fluorescent circline lamp from Sylvania, Product No. 20148, cool white phosphor, nominal wattage 22 W, color temperature 4200 K) at room temperature.

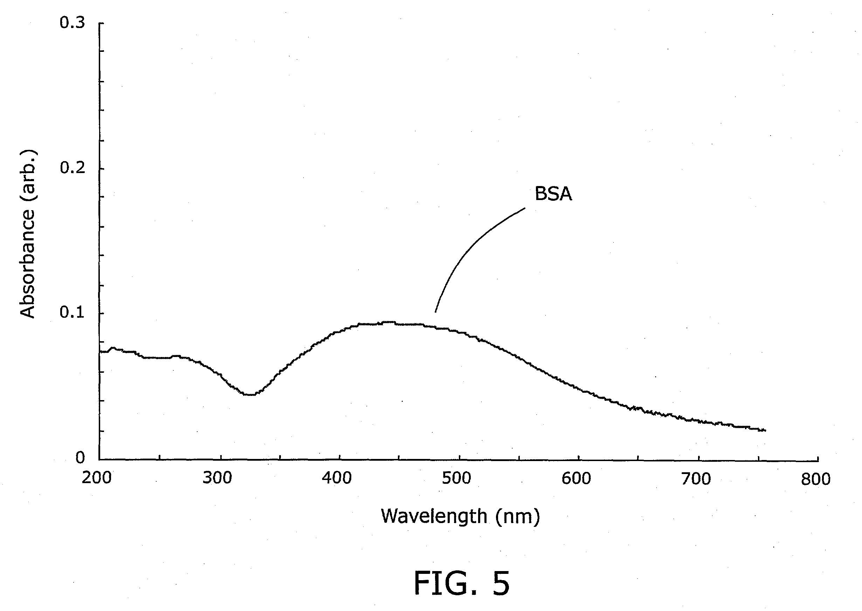

After 3 hours of irradiation the solution was examined. Already, the solution had adopted a reddish color that was visible to the naked eye. Figure 5 shows the absorption UV-visible absorption spectrum of the solution after 16 hours of irradiation. Typically, colloidal silver exhibits a visible absorption maximum (λₘₐₓ) in the range of about 405 nm to 410 nm (Bright et al., *Langmuir* 14:5695, 1998). Interestingly, the colloidal silver-BSA complexes prepared in this example exhibit a red-shifted λₘₐₓ in the visible region of 445 nm. It has been shown that λₘₐₓ for gold colloids tends to shift to higher wavelengths as the particles get larger and/or aggregate (Heath et al., *Phys. Rev. B: Condens. Matter* 40:9982, 1989 and Storhoff et al., *J. Am. Chem. Soc.* 122:4640, 2000). Similar results have been obtained with silver colloids (Bell and Myrick, *J. Colloid Interface Sci.* 242:300, 2001); however, the evidence is less clear since silver colloids tend to form less homogeneous collections of particles (Bright et al., *supra*).

### Example 2 - Preparing complexes using polynucleotides

The following describes the preparation and characterization of several polynucleotide complexes that were prepared using the inventive methods. Both single and double stranded polynucleotides of varying sizes were tested. The solutions listed in Table 2 were first prepared:

**TABLE 2**

| **Solution** | **Ingredient** | **Concentration (w/v)** |
|---|---|---|
| 1 | silver acetate | 0.2 % |
| 2a | poly(guanosine)² | 1 % |
| 2b | oligoDNA³ | 1% |
| 2c | dsDNA⁴ | 1% |
| 3 | zinc chloride | 1 % |

| | | |
|---|---|---|
| ¹Poly(guanosine) from Sigma-Aldrich. ²A synthetic 25-mer of DNA from Operon. | | |
| ³Double-stranded herring sperm DNA from Sigma-Aldrich. | | |

500 µl of solution 1 was added to 1 ml of deionized water in three 1.7 ml Eppendorf tubes. 5 µl of solutions 2a, 2b, and 2c were then added to one of the three tubes and these were vortexed to mix the ingredients. Finally, 5 µl of solution 3 was added to each tube, the resulting mixtures were vortexed, and then subjected to ambient irradiation of fluorescence light (Sylvania, Product No. 20148K) at room temperature.

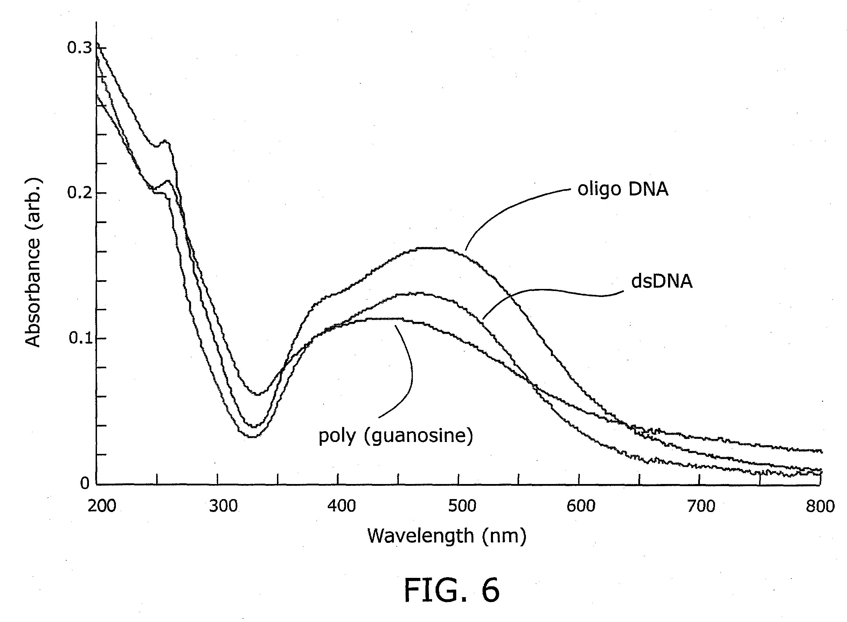

After 3 hours of irradiation the solutions were examined. Already, the solutions had adopted a reddish color that was visible to the naked eye. Figure 6 compares the absorption UV-visible absorption spectrum of the three solutions after 16 hours of irradiation. Interestingly, the colloidal silver-biomolecule complexes prepared in this example also exhibited a red-shifted λₘₐₓ in the visible region (Table 3).

**TABLE 3**

| Biomolecule | λₘₐₓ (nm) |
|---|---|
| poly(guanosine)² | 440 |
| oligoDNA³ | 480 |
| dsDNA⁴ | 470 |

| | |
|---|---|
| ¹Poly(guanosine) from Sigma-Aldrich. ²A synthetic 25-mer of DNA from Operon. | |
| ³Double-stranded herring sperm DNA from Sigma-Aldrich. | |

In order to ensure that colloidal silver-biomolecule complexes had been formed, the solutions were centrifuged at 15,000 rpm for 30 minutes on an Eppendorf microcentrifuge 5417C centrifuge from Brinkmann. The supernatants were then removed and the pellets re-suspended in deionized water. Absorption UV-visible absorption spectrum of the three solutions after re-suspension were then obtained (data not shown). Although the characteristic polynucleotide UV absorption peak (λₘₐₓ ~ 260 nm) was reduced as compared with the same peak prior to centrifugation, significant peaks were still present in all three cases confirming that amounts of the various polynucleotides were indeed stably associated with the colloidal silver particles.

### Example 3 - Varying the silver salt concentration

In order to investigate the effect of silver salt concentration on complex formation, the following experiments were performed. The solutions of Table 4 were first prepared:

**TABLE 4**

| **Solution** | **Ingredient** | **Concentration (w/v)** |
|---|---|---|
| 1 | silver acetate | 0.2 % |
| 2 | dsDNA¹ | 1 % |
| 3 | zinc chloride | 1 % |

| | | |
|---|---|---|
| ¹Double-stranded herring sperm DNA from Sigma-Aldrich. | | |

100 µl, 500 µl, and 1.5 ml of solution 1 was added to three separate 1.7 ml Eppendorf tubes. 1.4 ml and 1 ml of deionized water were added to the first and second tubes, respectively, in order to adjust the final volume to 1.5 ml. 5 µl of solution 2 was then added to each tube and the tubes were vortexed to mix the ingredients. Finally, 5 µl of solution 3 was added to each tube, the resulting mixtures were vortexed and then subjected to ambient irradiation of fluorescence light (Sylvania, Product No. 20148) at room temperature.

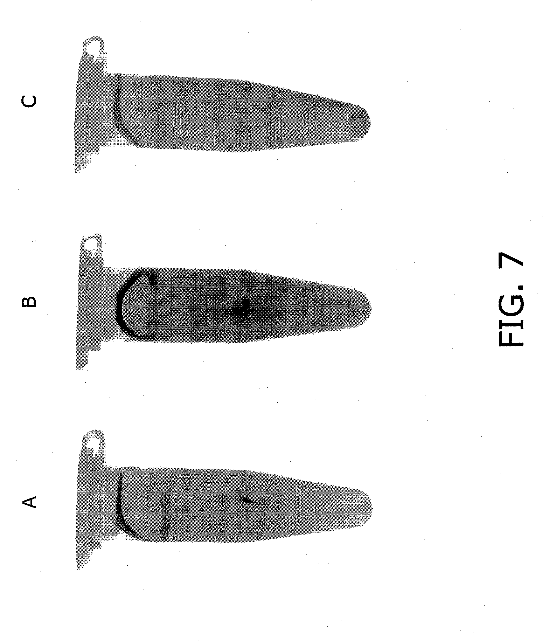

Figures 7A, 7B, and 7C show photographs taken of the first (x 0.2 silver salt), second (x 1 silver salt), and third (x 3 silver salt) solutions, respectively after 16 hours of irradiation. Figure 7C (increased silver salt) is not as dispersing as Figure 7B and includes a small amount of precipitate that does not show bright surface plasma absorbance suggesting that much larger particles have been formed. Figure 7A (decreased silver salt) is slightly less dispersing than Figure 7B and exhibits a lighter color.

### Example 4 - Varying the halide ion concentration

In order to investigate the effect of halide ion concentration on complex formation, the following experiments were performed.

The solutions of Table 4 were used. 500 µl of solution 1 was added to three separate 1.7 ml Eppendorf tubes and 1 ml of deionized water was added to each tube to adjust the final volume to 1.5 ml. 5 µl of solution 2 was then added to each tube and the tubes were vortexed to mix the ingredients. Finally, 0.5 µl, 5 µl, and 50 µl of solution 3 was added to each tube, the resulting mixtures were vortexed and then subjected to ambient irradiation of fluorescence light (Sylvania, Product No. 20148) at room temperature.

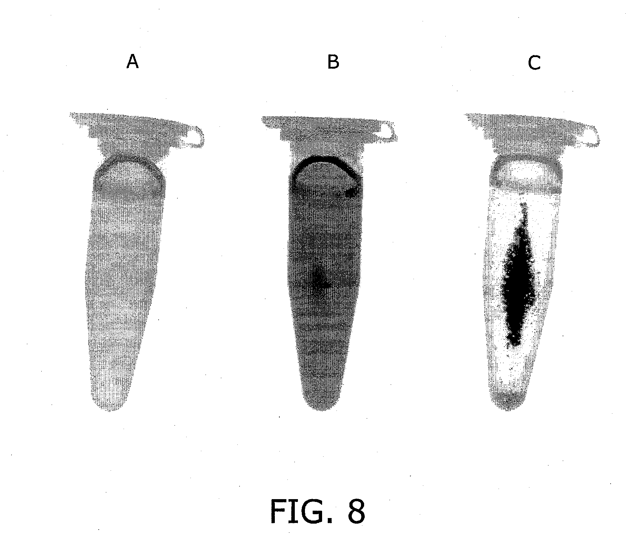

Figures 8A, 8B, and 8C compares photographs taken of the first (x 0.1 halide ion), second (x 1 halide ion), and third (x 10 halide ion) solutions, respectively after 16 hours of irradiation. Figure 8C (increased halide ion) shows a large amount of precipitation due to instant precipitation of silver halide after mixing. Figure 8A (decreased halide ion) exhibits a dispersed colloid solution but the color is much lighter than in Figure 8B implying that the halide ion is actively involved in the formation of the silver colloids and complexes.

### Example 5 - Varying the biomolecule concentration

In order to investigate the effect of biomolecule concentration on complex formation, the following experiments were performed.

The solutions of Table 4 were used. 500 µl of solution 1 was added to three separate 1.7 ml Eppendorf tubes and 1 ml of deionized water was added to each tube to adjust the final volume to 1.5 ml. 0.5 µl, 5 µl, and 50 µl of solution 2 was then added to the three tubes and the tubes were vortexed to mix the ingredients. Finally, 5 µl of solution 3 was added to each tube, the resulting mixtures were vortexed and then subjected to ambient irradiation of fluorescence light (Sylvania, Product No. 20148) at room temperature.

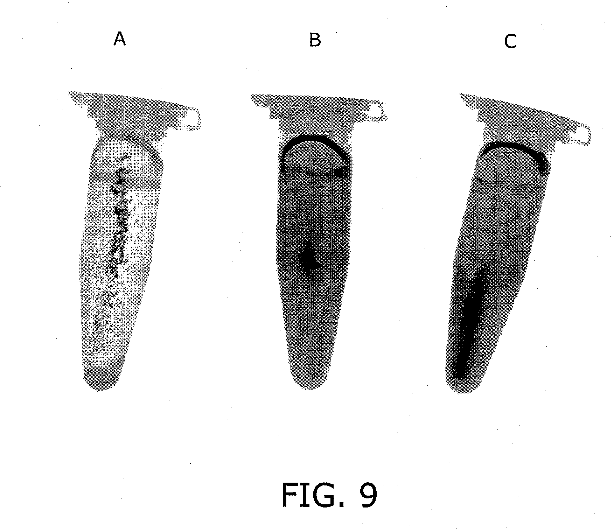

Figures 9A, 9B, and 9C compares photographs taken of the first (x 0.1 biomolecule), second (x 1 biomolecule), and third (x 10 biomolecule) solutions, respectively after 16 hours of irradiation. Figure 9C (increased biomolecule) though less dispersing also exhibits a silver colloid with comparable color as Figure 9B. Figure 9A (decreased biomolecule) exhibits a small amount of precipitate and no colloid implying that the biomolecule is actively participating in the formation of the silver colloids.

A control experiment was also performed in which solution 2 was replaced with a 1% (w/v) citric acid solution. Interestingly, no silver colloids or complexes were formed. Instead, the solution turned a brownish black color and large particles gradually precipitated within the tube (data not shown). This control experiment confirms that biomolecules are somehow playing an active role in stabilizing and/or promoting the formation of silver colloids and complexes.

### Example 6 - Use of other sources of halide ions

In order to investigate the effect of halide ion source on complex formation, the following experiments were performed. The solutions of Table 5 were first prepared:

**TABLE 5**

| **Solution** | **Ingredient** | **Concentration (w/v)** |
|---|---|---|
| 1 | silver acetate | 0.2 % |
| 2 | dsDNA¹ | 1% |
| 3a | potassium chloride | 1.1% |
| 3b | sodium chloride | 0.85 % |
| 3c | hydrogen chloride | 0.53 % |

| | | |
|---|---|---|
| ¹Double-stranded herring sperm DNA from Sigma-Aldrich. | | |

500 µl of solution 1 was added to three separate 1.7 ml Eppendorf tubes and 1 ml of deionized water was added to each tube to adjust the final volume to 1.5 ml. 5 µl of solution 2 was then added to the three tubes and the tubes were vortexed to mix the ingredients. Finally, 5 µl of solution 3a, 3b, or 3c was added to the three tubes, the resulting mixtures were vortexed and then subjected to ambient irradiation of fluorescence light (Sylvania, Product No. 20148) at room temperature.

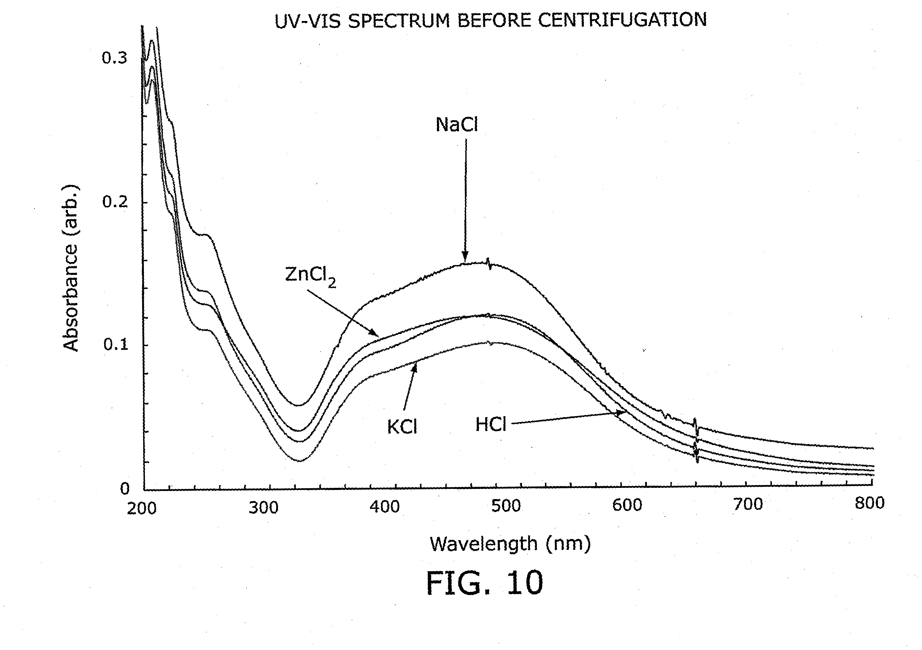

After 3 hours of irradiation the solutions were examined. Already, the solutions had adopted a reddish color that was visible to the naked eye. Figure 10 compares the absorption UV-visible absorption spectrum of complexes obtained with the zinc chloride (see Example 2), the potassium chloride, the sodium chloride, and hydrogen chloride solutions after 16 hours of irradiation.

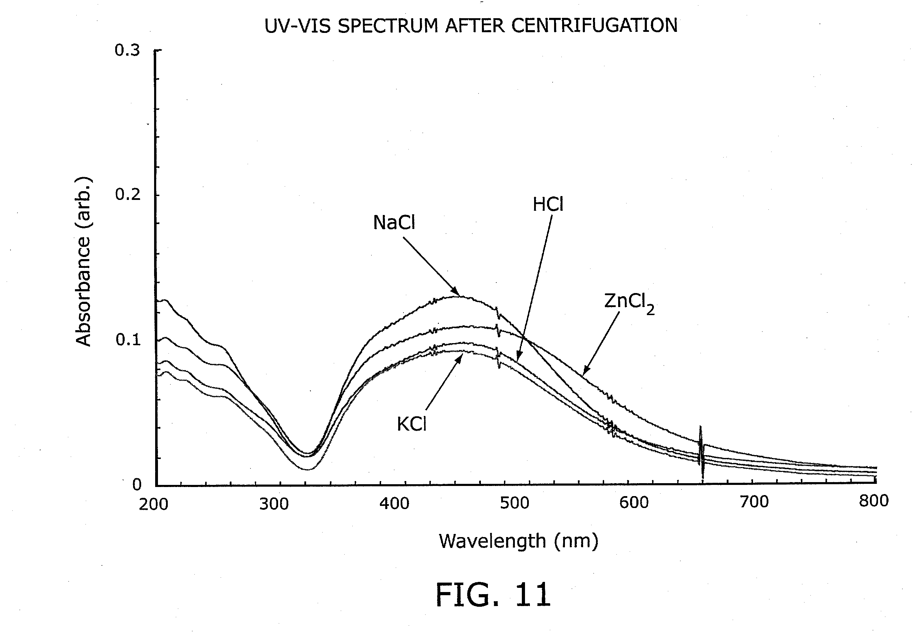

In order to ensure that a colloidal silver-dsDNA complex had been formed in each case, the solutions were centrifuged at 15,000 rpm for 30 minutes on an Eppendorf microcentrifuge 5417C centrifuge from Brinkmann. The supernatants were then removed and the pellets re-suspended in deionized water. Figure 11 compares the absorption UV-visible absorption spectra of the four solutions after re-suspension. Although the characteristic polynucleotide UV absorption peak (λₘₐₓ~ 260 nm) is reduced as compared with the same peak prior to centrifugation (see Figure 10), significant peaks were still present in all three cases confirming that amounts of the various dsDNA were indeed stably associated with the colloidal silver particles.

### Example 7 - Effect of irradiation

As a final control, the effect of irradiation on complex formation was analyzed by repeating the dsDNA preparation of Example 2 without exposing the solution to irradiation. No silver colloids or complexes were formed after a 16 hour period (data not shown).

### Other Embodiments

Other embodiments of the invention will be apparent to those skilled in the art from a consideration of the specification or practice of the invention disclosed herein. It is intended that the specification and examples be considered as exemplary only, with the true scope of the invention being indicated by the following claims.

## Claims

1. A method for preparing a colloidal silver-biomolecule complex comprising:
providing a mixture of a biomolecule, a silver salt, and a source of halide ions in a single solution; and
irradiating the mixture with light having a wavelength in the visible region, wherein
the silver salt and source of halide ions are water soluble;
the amounts of the biomolecule, the silver salt and the source of halide ions being such that, the irradiating step results in formation of colloidal silver-biomolecule complexes.

2. A method as claimed in claim 1, wherein:
the biomolecule comprises a polynucleotide or a polypeptide;
the silver salt is silver acetate, silver benzoate, silver bromate, silver chlorate, silver perchlorate, silver chlorite, silver fluoride, silver lactate, silver levunilate, silver permanganate, silver nitrate, silver nitrite, silver propionate, silver sulfate, silver tartrate, or any hydrates thereof;
the source of halide ions is a Group IA metal chloride, bromide, iodide, or a hydrate thereof; a Group IIA metal chloride, bromide, iodide, or a hydrate thereof; a transition metal chloride, bromide, iodide, or a hydrate thereof, with the proviso that the source of halide ions is not a silver halide or a mercury (I) halide; or zinc chloride, potassium chloride, or sodium chloride.

3. A colloidal silver-biomolecule complex obtainable by means of a method as claimed in claim 1 or 2.

4. A solution comprising a colloidal silver-biomolecule complex, wherein the solution further comprises halide ions and absorbs light in the visible region with a wavelength of maximum absorption in the range from about 420 nm to 500 nm, preferably from about 430 nm to 450 nm, most preferably from about 460 nm to 480 nm.

5. A solution as claimed in claim 4, wherein the biomolecule comprises a polynucleotide or a polypeptide.

6. A kit comprising:
a silver salt;
a source of halide ions, wherein the silver salt and the source of halide ions are packaged together with instructions for combining them together in a solution with a biomolecule to form a mixture **characterised in that**, when the mixture is irradiated with light having a wavelength in the visible region, colloidal silver-biomolecule complexes form, the kit optionally further comprising a biomolecule.

7. A method for determining the presence of a biomolecule in sample, the method comprising steps of:
mixing in solution a sample that includes a biomolecule with a silver salt and a source of halide ions;
irradiating the mixture with light having a wavelength in the visible region;
detecting the formation of colloidal silver-biomolecule complexes in the solution; and
determining the presence of the biomolecule in the sample based on the step of detecting, optionally wherein the step of detecting is carried out by:
observing the mixture using the naked eye;
observing the mixture using microscopy;
measuring an optical absorption of the mixture; or
measuring surface-enhanced Raman scattering from biomolecules within colloidal silver-biomolecule complexes.

8. A method comprising steps of:
providing a substrate that is associated on its surface with an array of separate and different molecular elements, wherein molecules within different molecular elements of the array include binding complements for different biomolecules;
contacting the substrate with a sample that includes one or more colloidal silver-target biomolecule complexes as claimed in claim 3 so that the binding complements in the array can interact and bind with biomolecules within complexes; and
removing complexes that are not associated with the substrate by washing, the method optionally further comprising:
identifying the location of complexes that are associated with the substrate; and
determining the nature of one or more biomolecules in the sample based on the step of identifying.

9. A method for detecting surface-enhanced Raman scattering from a biomolecule, the method comprising steps of:
providing a colloidal silver-target biomolecule complex as claimed in claim 3; and
measuring surface-enhanced Raman scattering from the biomolecule within the colloidal silver-biomolecule complex.

10. A method comprising steps of:
providing one or more colloidal silver-target biomolecule complexes as claimed in claim 3; and
manipulating the one or more of the complexes using optical tweezers.
